# EUROPÄISCHE PATENTSCHRIFT

(11) **EP 1 372 685 B1**
(45) Veröffentlichungstag und Bekanntmachung des Hinweises auf die Patenterteilung: **29.04.2009**
(21) Anmeldenummer: 02726220.3
(22) Anmeldetag: 27.03.2002
(51) Int. Cl.: A61K 36/77, A61K 8/97, A61Q 17/04, A61Q 19/08, A61P 39/06, A61P 29/00, A61P 17/00

(54) **VERWENDUNG VON EXTRAKTEN DER PFLANZE LITCHI CHINENSIS SONN.**
USE OF EXTRACTS OF THE PLANT LITCHI CHINENSIS SONN.
UTILISATION D'EXTRAITS DE LA PLANTE LITCHI CHINENSIS SONN.

(30) Priorität: 03.04.2001 EP 01400844
(43) Veröffentlichungstag der Anmeldung: 02.01.2004
(73) Patentinhaber: Cognis France, S.A.S., 31360 Boussens (FR)
(72) Erfinder: PAULY, Gilles, F-54000 Nancy (FR); DANOUX, Louis, F-54420 Saulxures les Nancy (FR); HENRY, Florence, F-54600 Villers-les-Nancy (FR)
(74) Vertreter: Fabry, Bernd
(86) Internationale Anmeldenummer: PCT/EP2002/003426
(87) Internationale Veröffentlichungsnummer: WO 2002/080949

(56) Entgegenhaltungen:
- PATENT ABSTRACTS OF JAPAN vol. 017, no. 025 (C-1017), 18. Januar 1993 (1993-01-18) & JP 04 247008 A (MIKIMOTO SEIYAKU KK), 3. September 1992 (1992-09-03)
- PATENT ABSTRACTS OF JAPAN vol. 017, no. 025 (C-1017), 18. Januar 1993 (1993-01-18) & JP 04 247009 A (MIKIMOTO SEIYAKU KK), 3. September 1992 (1992-09-03) in der Anmeldung erwähnt
- PATENT ABSTRACTS OF JAPAN vol. 2000, no. 08, 6. Oktober 2000 (2000-10-06) & JP 2000 128730 A (ICHIMARU PHARCOS CO LTD), 9. Mai 2000 (2000-05-09)
- SARNI-MACHADO P. ET AL: 'Phenolic Composition of Litchi Fruit Pericarp COMPOSITION OF LITCHI FRUIT pERICARP' J.AGRIC.FOOD CHEM. Bd. 48, 2000, Seiten 5995 - 6002, XP002259112

## Beschreibung

### Gebiet der Erfindung

Die Erfindung befindet sich auf dem Gebiet der kosmetischen Verwendung von Extrakten aus der Fruchthülle der Pflanze Litchi chinensis Sonn.

### Stand der Technik

Kosmetische Zubereitungen stehen dem Verbraucher heute in einer Vielzahl von Kombinationen zur Verfügung. Dabei wird nicht nur erwartet, daß diese Kosmetika einen bestimmten pflegenden Effekt zeigen oder einen bestimmten Mangel beheben, sondern immer häufiger wird nach Produkten verlangt, die mehrere Eigenschaften gleichzeitig aufweisen und somit ein verbessertes Leistungsspektrum zeigen. Ebenso darf der Anwender erwarten, daß die Zusammensetzung des Produktes eine optimale dermatologische Verträglichkeit besitzt, so daß auch empfindliche Verbraucher nicht mit Irritationen reagieren. Darüber hinaus sollten die Mittel jedoch auch weitere Funktionen erfüllen, die zunehmend im Bereich der Pflege und insbesondere dem Schutz von Haut und Haar liegen.

Extrakte von Pflanzen und deren Inhaltsstoffe finden immer häufiger Einsatz in der Kosmetik und Dermatologie. Pflanzenextrakte werden seit vielen Jahren in den unterschiedlichsten Kulturen für medizinische aber auch bereits für kosmetische Zwecke genutzt.

So beschreiben JP4247008 und JP4247009 einem Extrakt aus Litchichinensis Sonnerat Früchten bzw. eine Mischung von zwei Pflanzenextrakten aus der Frucht von Litchi chinensis Sonn. und den ganzen Pflanzenteilen von Ganoderma lucidum Karst., die mittels eines wasserlöslichen organischen Lösungsmittels extrahiert wurden. Die erhaltene Zusammensetzung wird als Skin whitener und Feuchtigkeitsspender verwendet.

Weiterhin wird in Sarni-Manchado et al. J. Agric. Food Chem., 2000, 48, 5995-6002 die chemishe Zusammensetzung von Fruchthüllen aus Litchi chinensis analysiert.

### Beschreibung der Erfindung

Die Aufgabe der vorliegenden Patentanmeldung hat darin bestanden, Extrakte aus nachwachsenden Rohstoffen für die kosmetische und/oder dermatologische Anwendung zur Verfügung zu stellen, die einen Einsatz in der Kosmetik ermöglichen und neben pflegenden Eigenschaften vor allem verbesserte schützende Eigenschaften für menschliche Haut und/oder Haare beispielsweise gegen UV-Strahlung und andere Umwelteinflüsse besitzen und gleichzeitig vorbeugende und heilende Wirkung bei Alterserscheinungen der Haut zeigen und antiinflammatorisch einsetzbar sind.

Eine weitere Aufgabe der vorliegenden Patentanmeldung hat darin bestanden, Zubereitungen zur Verfügung zu stellen, die Wirkstoffe aus nachwachsenden Rohstoffen enthalten und gleichzeitig vielseitig als Pflegemittel in der Kosmetik sowohl in der Hautkosmetik, als auch in der Haarpflege einsetzbar sind.

Diese mehrfachen Einsatzgebiete der zu verwendenden Mittel aus dem nachwachsenden Rohstoff der Fruchthülle der Pflanze Litchi chinensis Sonn. (im weiteren als Litchi-Extrakt bezeichnet) macht es für den Markt und für den Verbraucher sehr attraktiv. Die komplexe Aufgabe der Erfindung konnte somit durch die kosmetische Verwendung eines Extraktes der Fruchthüller der Pflanze Litchi chinensis Sonn. gelöst werden wobei die Extrakte Flavonderivate enthalten, die ausgewählt sind aus der Gruppe, die gebildet wird von Flavanen, Flavan-3-olen, Flavan-3,4-diolen, Flavonen, Flavonolen, Flavononen und deren Derivate. Die darin enthaltenen Procyanidolischen Oligomere (OPC) werden vorzugsweise zur Erhöhung der Stabilität derivatisiert.

Unter dem Begriff Pflanze im Sinne der vorliegenden Anmeldung sind sowohl ganze Pflanzen als auch Pflanzenteile (Blätter, Wurzeln, Blüten) sowie deren Gemische zu verstehen.

### Litchi chinensis Sonn.

Die zur kosmetischen Verwendung eingesehten Extrakte werden aus Pflanzen der Gattung Sapindaceae, speziell aus der Art Litchi chinensis Sonn. gewonnen. Litchis sind unter guten Bedingungen bis zu 10 m Höhe erreichende, langsam wachsende Bäume mit einem grauen Stamm. Die Blätter sind ledrig und relativ dick. Neue Blätter sind zuerst rötlich und ändern erst mit der Zeit ihre Farbe nach sattgrün. Die kleinen, gelbgrünen Blüten bilden lange, oft nach oben stehende Rispen. Ihre Heimat liegt in Süd-China, die Pflanzen werden jedoch mittlerweile weltweit kultiviert.

Die reife Frucht hat eine dunkelrot-braune, rauhe Oberfläche und eine Größe von 2,5 - 4 cm. Unter der brüchigen Fruchthülle besitzt sie weißschimmemdes Fruchtfleisch mit süßem Geschmack und geleeartiger aber fester Konsistenz. Der Kern der Litchi nimmt einen bedeutenden Anteil der gesamten Frucht ein und ist kastanienbraun gefärbt.

### Extraktion

Die Herstellung der einzusetzenden Extrakte erfolgt durch übliche Methoden der Extraktion der Fruchthülle der Pflanze. Als Ausgangsmaterial können frische oder getrocknete Fruchthüllen eingesetzt werden, üblicherweise wird jedoch von Fruchthüllen ausgegangen, die vor der Extraktion mechanisch zerkleinert und gegebenenfalls entfettet werden.

Als Lösungsmittel für die Durchführung der Extraktionen können vorzugsweise organische Lösungsmittel, Wasser oder Gemische aus organischen Lösungsmitteln und Wasser, insbesondere niedermolekulare Alkohole, Ester, Ether, Ketone oder halogenhaltige Kohlenwasserstoffe mit mehr oder weniger hohen Wassergehalten (destilliert oder nicht destilliert) vorzugsweise wässrig, alkoholische Lösungen mit mehr oder weniger hohen Wassergehalten, verwendet werden. Besonders bevorzugt ist die Extraktion mit Methanol, Wasser, Ethanol sowie Mischungen hieraus. Die Extraktion erfolgt in der Regel bei 20 bis 100 °C, bevorzugt bei 30 bis 90°C, insbesondere bei 40 bis 60 °C. In einer möglichen Ausführungsform erfolgt die Extraktion unter Inertgasatmosphäre zur Vermeidung der Oxidation der Inhaltsstoffe des Extraktes. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt. Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, unterzogen werden. Die Extraktion kann bis zu jedem gewünschten Extraktionsgrad erfolgen, wird aber gewöhnlich bis zur Erschöpfung durchgeführt. Typische Ausbeuten (= Trockensubstanzmenge des Extraktes bezogen auf eingesetzte Rohstoffmenge) bei der Extraktion getrockneter Pflanzen oder getrockneter Pflanzenteile gegebenenfalls entfettet, liegen im Bereich von 5 bis 20 Gew.%. Die vorliegende Erfindung umfasst die Erkenntnis, dass die Extraktionsbedingungen sowie die Ausbeuten der Endextrakte je nach gewünschtem Einsatzgebiet gewählt werden können. Falls gewünscht, können die Extrakte anschließend beispielsweise einer Sprüh- oder Gefriertrocknung unterworfen werden.

Die Einsatzmenge der Pflanzenextrakte in den genannten Zubereitungen richtet sich nach der Konzentration der einzelnen Inhaltstoffe und nach der Art der Anwendungen der Extrakte. Die

Gesamtmenge des Pflanzenextraktes, der in den zu verwendenden Zubereitungen enthalten ist, beträgt in der Regel 0,001 bis 25 Gew.%, vorzugsweise 0,01 bis 5 Gew.%, insbesondere 0,05 bis 1,5 Gew.% bezogen auf die Endzubereitung, mit der Maßgabe, dass sich die Mengenangaben mit Wasser und gegebenenfalls weiteren Hilfs- und Zusatzstoffen zu 100 Gew.% addieren.

Die zu verwendenden Extrakte enthalten einen Wirkstoffgehalt in den Extrakten von 5 bis 100 Gew.%, vorzugsweise 10 bis 95 Gew.%, insbesondere 20 bis 80 Gew.%. Der Wirkstoffgehalt im Sinne der Erfindung bezeichnet die Summe aller im Extrakt vorhandenen Wirkstoffe bezogen auf das Trockengewicht des Extraktes.

Wirkstoff im Sinne der Erfindung bezieht sich auf die im Extrakt enthaltenen Inhaltstoffe auch wenn deren Gehalt und Identität mit herkömmlichen, dem Fachmann bekannten Methoden noch nicht nachzuweisen sind. Unter Wirkstoffe im Sinne der Erfindung sind weiterhin alle im Extrakt erhaltenen Inhaltsstoffe zu verstehen, deren Wirkung entweder bereits bekannt ist, oder deren Wirkung mit herkömmlichen, dem Fachmann bekannten Methoden noch nicht nachgewiesen werden konnte.

Aktivsubstanz im Sinne der Erfindung bezieht sich auf den Anteil an Substanzen sowie Hilfs- und Zusatzstoffen, die in dem Mittel enthaltend sind, mit Ausnahme des zusätzlich hinzugefügten Wassers. Der Gesamtanteil an Hilfs- und Zusatzstoffe kann 1 bis 50, vorzugsweise 5 bis 40 Gew.% - bezogen auf die Endzubereitung der kosmetischen und/oder dermatologischen Zubereitungen - betragen. Die Herstellung der Zubereitungen kann durch übliche Kalt - oder Heißprozesse erfolgen; vorzugsweise arbeitet man nach der Phaseninversionstemperatur-Methode.

### Extrakte

Die zu verwendenden Extrakte der Fruchthülle der Pflanze Litchi chinensis Sonn. enthalten in der Regel Inhaltsstoffe aus der Gruppe bestehend aus Flavonderivaten, insbesondere Flavanolen, Anthocyaninen und Flavonolen.

Im Sinne der vorliegenden Erfindung sind unter Flavonderivaten zu verstehen: Flavane, Flavan-3-ole (Catechine, Catechin-Oligomere), Flavan-3,4-diole (Leukoanthocyanidine), Elavone, Flavonole und Flavonone sowie deren Derivate.

Als Flavonderivate isoliert aus der Fruchthülle der Pflanze Litchi chinensis Sonn. gelten die Procyanidolischen Oligomere (OPC). Dabei handelt es sich um Oligomere von 2 bis 8 Monomereinheiten des Catechins und/oder Epicatechins. Die Proanthocyanidolischen Oligomere (OPC) werden wegen ihrer Vitamin P-Aktivität geschätzt.

Zur Erhöhung der Stabilität in Formulierungen werden die Flavone vorzugsweise nach Extraktion derivatisiert und die erhaltenen Derivate in den Formulierungen eingesetzt. Als besonders bevorzugt gelten hier die Ester.

Ein Gegenstand der Erfindung ist die kosmetische Verwendung von Extrakten aus der Fruchthülle der Pflanze Litchi chinensis Sonn. als Pflegemittel für Haut und/oder Haar. wobei die Extrakte Flavonderivate enthalten, die ausgewählt sind aus der Gruppe, die gebildet wird von Flavanen, Plavan-3₋olen, Flavan-3,4-diolen, Flavonen, Flavonolen, Flavononen und deren Derivate.

Die Verwendung hat kosmetische Wirkung.

### Pflegemittel:

Als Pflegemittel im Sinne der Erfindung sind Pflegemittel für Haut und Haar zu verstehen. Diese Pflegemittel schließen unter anderem reinigende und aufbauende Wirkung für Haut und Haare ein.

Die Applikation kann sowohl topisch als auch oral in Form von Tabletten, Dragees, Kapseln, Säfte, Lösungen und Granulate erfolgen.

Die zu verwendenden Zubereitungen zeigen darüber hinaus eine hervorragende hautpflegende Wirkung bei gleichzeitig hoher Hautverträglichkeit. Die Zubereitungen weisen eine Vielzahl von Kosmetischen Wirkungen auf. Weitere Gegenstände der Erfindung betreffen daher die kosmetische Verwendung von Extrakten aus der Fruchthülle der Pflanze Litchi chinensis Son. wobeidie Extrakte Flavonderivate enthalten, die ausgewählt sind aus der Gruppe, die gebildet wird von Flavanen, Flavan-3-olen, Flavan-3,4-diolen, Flavonen, Flavonolen, Flavononen und deren Derivate.
➢ als Sonnenschutzmittel; insbesondere gegen UVA-Strahlung und/oder gegen UVB-Strahlung;
➢ gegen freie Radikale;
➢ als Antioxidans;
➢ als anti-inflammatorische Mittel, insbesondere zur Inhibierung der schädlichen Effekte von "respiratory burst" aufgrund des Durchdringens von Polymorphonuclearen Leukocyten in gestresster Haut.
➢ als Mittel gegen die Hautalterung
➢ als protease-inhibierendes Mittel, insbesondere als Plasmin(Serin-Protease)-inhibierendes Mittel und/oder als MMP- und/oder Collagenase- und/oder Elastase-inhibierendes Mittel;

Unter dem Begriff "respiratory burst" wird im Sinn der Erfindung die Aktivierung von Leukozyten, speziell polymorphonucleare neutrophile Granulocyten verstanden.

Eine kutane Inflammation (Entzündung) kann hervorgerufen werden durch UVB Strahlung aufgrund der Stimulierung epidermaler Keratinocyten. Anschließend setzt eine akute Leukozyten Infiltration ein.

Diese Aktivierung dieser Leukozyten, speziell polymorphonucleare neutrophile Granulocyten, ist bekannt als "respiratory burst" und kann eine Gewebezerstörung vermitteln durch freigesetzten reaktiven Sauerstoffradikalen (reactive oxygen species - ROS) und durch lysosomale Enzyme.

### Sonnenschutzmittel bzw. UV-Lichtschutzfaktoren

Die Extrakte aus der Fruchthülle der Pflanze Litchi chinensis Sonn. wirken im Sinne der Erfindung als Sonnenschutzmittel. Bevorzugt sind hierbei die Extrakte, die obengenannten Flavonderivate enthalten.

Als Sonnenschutzmittel bzw. UV-Lichtschutzfaktoren im Sinne der Erfindung werden Lichtschutzmittel bezeichnet, die für den Schutz der menschlichen Haut gegenüber schädigenden Einflüssen der direkten und indirekten Strahlung der Sonne nützlich sind. Die für die Hautbräunung verantwortliche Ultraviolettstrahlung der Sonne unterteilt man in die Abschnitte UV-C (Wellenlängen 200-280 nm), UV-B (280-315 nm) u. UV-A (315-400 nm).

Die Pigmentierung normaler Haut unter dem Einfluss der Sonnenstrahlung, d. h. die Bildung von Melaninen, wird durch UV-B u. UV-A unterschiedlich bewirkt. Bestrahlung mit UV-A-Strahlen ("langwelligem UV") hat die Dunkelung der in der Epidermis bereits vorhandenen Melanin-Körper zur Folge, ohne dass schädigende Einflüsse zu erkennen sind. Anders bei dem sog. "kurzwelligen UV" (UV-B). Dieses bewirkt die Entstehung von sog. Spätpigment durch Neubildung von Melanin-Kömem. Ehe jedoch das (schützende) Pigment gebildet ist, unterliegt die Haut der Einwirkung der ungefilterten Strahlung, die - je nach Expositionsdauer - zur Bildung von Hautrötungen (Erythemen), Hautentzündungen (Sonnenbrand) u. gar Brandblasen führen kann.

Extrakte aus der Fruchthülle der Pflanze Litchi chinenesis Sonn. können zusätzlich in Kombination mit Sonnenschutzmitteln bzw. UV-Lichtschutzfaktoren, welche als UV-Absorber oder Lichfilter die UV-Strahlung in unschädliche Wärme umwandeln, vorliegen.

Diese weiteren UV- Lichtschutzfaktoren sind beispielsweise bei Raumtemperatur flüssig oder kristallin vorliegende organische Substanzen (Lichtschutzfilter), die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
➢ 3-Benzylidencampher bzw. 3-Benzylidennorcampher und dessen Derivate, z.B. 3-(4-Methylbenzyliden)campher wie in der EP 0693471 B1 beschrieben;
➢ 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
> Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-isopropylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexylester;
➢ Triazinderivate, wie z.B. 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der EP 0694521 B1 beschrieben.

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ Sulfonsäurederivate von Benzophenonen, vorzugsweise 2-Hydroxy-4-methoxybenzophenon-5-sulfonsäure und ihre Salze;
➢ Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UVA-Filter kommen insbesondere Derivate des Benzoylmethans in Frage, wie beispielsweise 1-(4'-tert.Butylpheny)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beispielsweise beschrieben in der DE 19712033 A1 (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Dimethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 543 (1996**)** sowie Parfümerie und Kosmetik 3 (1999), Seite 11ff zu entnehmen.

Die Extrakte aus der Fruchthülle der Pflanze Litchi chinensis Sonn. wirken im Sinne der Erfindung gegen die Schädigung von Fibroblasten und/oder Keratinocyten durch UVA-Strahlung und/oder UVB-Strahlung.

UVA-Strahlen dringen bis in die Dermis ein, wo sie zu Oxidationsstreß führen, was durch eine Lipoperoxidation der Zytoplasmamembranen nachgewiesen wird. Die Lipoperoxide werden zu Malonaldialdehyd (MDA) abgebaut, der viele biologische Moleküle wie Proteine und Nukleinbasen vernetzen wird (Enzymhemmung bzw. Mutagenese). Die zu verwendenden Extrakte aus der Fruchthülle der Pflanze Litchi chinensis Sonn reduzieren signifikant den Grad an MDA in humanen Fibroblasten, welcher durch UVA-Strahlen induziert wird und zeigen damit eine hohe Kapazität schädliche Effekte eines oxidativen Stresses auf der Haut zu reduzieren.

UVB-Strahlen lösen durch Aktivierung eines Enzyms, nämlich Phospholipase A2 oder PLA2 eine Entzündung aus. Diese Entzündung (Erythem, Ödem) wird durch die Entfernung von Arachidonsäure aus den Phospholipiden der Plasmamembran durch die Phospholipase ausgelöst. Arachidonsäure ist die Vorstufe der Prostaglandine, die eine Entzündung und eine Zellmembranschädigung verursachen; die Prostaglandine E2 (= PGE2) werden durch die Cyclooxygenase gebildet. Der Grad der Freisetzung des Cytoplasaenzyms LDH (Lactat Dehydrogenase) in humanen Keratinocyten dient als Marker für eine Zellschädigung.

Die zu verwendenden Extrakte aus der Fruchthülle der Pflanze Litchi chinensis Sonn. reduzieren den Effekt von UVB-Strahlung auf die Anzahl an Keratinocyten und auf den Gehalt an freigesetzte LDH. Die Extrakte zeigen demnach die Fähigkeit, die durch UVB-Strahlung hervorgerufene Schädigung an Zellmembranen zu reduzieren.

Die Extrakte aus der Fruchthülle der Pflanze Litchi chinensis Sonn. wobei die Extrakte Flavonderivate enthalten, die ausgewählt sind aus der Gruppe, die gebildet wird von Flavanen, Flavan-3-olen, Flavan-3,4-diolen, Flavonen, Flavonolen, Flavononen und deren Derivate, wirken im Sinne der Erfindung als Antioxidans bzw Radikalfänger.

Als Antioxidantien im Sinne der Erfindung sind Oxidationsinhibitoren zu verstehen, die sich aus der Fruchthülle der Pflanze Litchi chinensis Sonn isolieren lassen. Antioxidantien sind in der Lage, die unerwünschten, durch Sauerstoff-Einwirkungen und andere oxidative Prozesse bedingten Veränderungen in den zu schützenden Stoffen zu hemmen oder zu verhindern. Die Wirkung der Antioxidantien besteht meist darin, dass sie als Radikalfänger für die bei der Autoxidation auftretenden freien Radikale wirken.

Neben der Verwendung von Extrakten der Fruchthülle der Pflanze Litchi chinensis Sonn. als Antioxidantien können auch weitere, bereits bekannte Antioxidantien eingesetzt werden. Eine mögliche Anwendung der Antioxidantien in kosmetischen Zubereitungen, ist die Anwendung als sekundäre Lichtschutzmittel, weil Antioxidantien in der Lage sind, die photochemische Reaktionskette zu unterbrechen, welche ausgelöst wird, wenn UV-Strahlung in die Haut eindringt Neben dem zu verwendenden Pflanzenextrakt sind weitere typische Beispiele hierfür Aminosäuren (z.B. Glycin, Alanin, Arginin, Serin, Threonin, Histidin, Tyrosin, Tryptophan) und deren Derivate, Imidazole (z.B. Urocaninsäure) und deren Derivate, Peptide wie D,L-Carnosin, D-Camosin, L-Camosin und deren Derivate (z.B. Anserin), Carotinoide, Carotine (z.B. α-Carotin, β-Carotin, Lycopin, Lutein) oder deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, Boldin, Boldo-Extrakt, EDT, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die weiteren UV-Lichtschutzfaktoren bzw. Antioxidantien können in Mengen von 0,01 bis 25, vorzugsweise 0,03 bis 10 und insbesondere 0,1 bis 5 Gew.-% bezogen auf die Gesamtmenge in den Zubereitungen, zugegeben werden.

Die Extrakte aus der Fruchthülle der Pflanze Litchi chinensis Sonn., welche Flavonderivate enthalten, die ausgewählt sind aus der Gruppe von Flavanen, Flavan-3-olen, Flavan-3,4-diolen, Flavonen, Flavonolen, Flavononen und deren Derivaten, wirken im Sinne der Erfindung als anti-inflammatorisches Pflegemittel, die eine Entzündung der Haut heilen können oder die einer Entzündung vorbeugen können. Die Entzündungen können dabei die unterschiedlichsten Ursachen aufweisen. Insbesondere können Entzündungen behandelt werden, die durch UV-Strahlung, Hautverunreinigungen oder bakteriell wie hormonell-bedingte Hautveränderungen, z. B. Akne induziert werden.

Die oben definierten Extrakte aus der Fruchthülle der Pflanze Litchi chinensis Sonn. wirken im Sinne der Erfindung gegen Hautalterungen, insbesondere gegen jede Art der Fältchen- und Faltenbildung. Eine andere Bezeichnung für diese Art der Pflegemittel ist auch anti-ageing Mittel. Die Verwendungen schließen eine Verlangsamung von Altersprozessen der Haut mit ein. Die Alterserscheinungen können die unterschiedlichsten Ursachen aufweisen. Insbesondere können diese Alterserscheinungen auf Grund von Apoptose, durch UV-Strahlung oder durch die Zerstörung der hauteigenen Proteine wie beispielswe Collagen oder Elastin induzierten Schädigungen der Haut verursacht sein. Die oben definierten Extrakte aus der Fruchthülle der Pflanze Litchi chinensis Sonn. wirken als Protease-inhibierendes Mittel, insbesondere als Plasmin und/oder MMP- und/oder Collagenase- und/oder Elastase-inhibierendes Mittel. Unter MMP versteht man Matrix-Metallo-Proteasen.

Die Verwendung der oben definierten Extrakte als schützende und aufbauende Pflegemittel ist prinzipiell für alle Zubereitungen, möglich, die zur Prävention gegen Schädigungen oder bei Schädigungen der Haut und/oder Haare und damit in der Haut- und Haarpflege eingesetzt werden. Eine andere Verwendung auf diesem Gebiet ist die Applikation bei empfindlicher, durch Allergie oder anderen Ursachen geschädigter Haut. Die Schädigung der Haut kann dabei unterschiedlichste Ursachen haben.

Die oben definierten Extrakte können zur Herstellung von kosmetischen Zubereitungen, wie beispielsweise Schaumbäder, Duschbäder, Cremes, Gele, Lotionen, alkoholische und wäßrig/alkoholische Lösungen, Emulsionen, Wachs/ Fett-Massen, Stiftpräparaten, Pudern oder Salben zum Einsatz kommen.

Diese Zubereitungen können ferner als weitere Hilfs- und Zusatzstoffe milde Tenside, Ölkörper, Emulgatoren, Periglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. amphotere Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quatemierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise J.Falbe (ed.), "Surfactants in Consumer Products", Springer Verlag, Berlin, 1987, S. 54-124 oder J.Falbe (ed.), "Katalysatoren, Tenside und Mineralöladditive", Thieme Verlag, Stuttgart, 1978, S. 123-217 verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen (vgl. DE 19756377 A1), insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestem mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
➢ Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
➢ Alkyl- und/oder Alkenyloligoglykoside mit 8 bis 22 Kohlenstoffatomen im Alk(en)ylrest und deren ethoxylierte Analoga;
➢ Anlagerungsprodukte von 1 bis 15 Mol Ethylenoxid an Ricinusöl und/oder gehärtetes Ricinusöl;
➢ Anlagerungsprodukte von 15 bis 60 Mol Ethylenoxid an Ricinusöl und/oder gehärtetes Ricinusöl;
➢ Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Mischester aus Pentaerythrit, Fettsäuren, Citronensäure und Fettalkohol gemäß DE 1165574 **PS** und/oder Mischester von Fettsäuren mit 6 bis 22 Kohlenstoffatomen, Methylglucose und Polyolen, vorzugsweise Glycerin oder Polyglycerin.
➢ Mono-, Di- und Trialkylphosphate sowie Mono-, Di- und/oder Tri-PEG-alkylphosphate und deren Salze;
➢ Wollwachsalkohole;
➢ Polysiloxan-Polyalkyl-Polyether-Copolymere bzw. entsprechende Derivate;
➢ Block-Copolymere z.B. Polyethylenglycol-30 Dipolyhydroxystearate;
➢ Polymeremulgatoren, z.B. Pemulen-Typen (TR-1,TR-2) von Goodrich;
➢ Polyalkylenglycole sowie
➢ Glycerincarbonat..

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht C_{12/18}-Fettsäuremono- und -diester von Anlagerungsprodukten von Ethylenoxid an Glycerin sind aus DE 2024051 **PS** als Rückfettungsmittel für kosmetische Zubereitungen bekannt.

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckenest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete Partialglyceride sind Hydroxystearinsäuremonoglycerid, Hydroxystearinsäuredigiycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurndiglycerid, Citronensäuremonoglycerid, Citronendiglycerid, Äpfelsäuremonoglycerid, Äpfefsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester. '

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolane Gl 34), Polyglyceryl-3 Oleate, Diisostearoyl Polyglyceryl-3 Diisostearate (isolano PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010190), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Olsäure, Behensäure und dergleichen.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispiels-weise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquatemierte Difettsäuretriethanolaminester Salze, besonders bevorzugt sind.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u,a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligogluooside sowie Elektrolyte wie Kochsalz und Ammoniumchlorid.

### Überfettungsmittel

Als Oberfettungsmittel können Substanzen wie beispielsweise Lanolin und Lecithin sowie polyethoxy lierte oder acylierte Lanolin- und Lecithinderivate, Polyolfettsäureester, Monoglyceride und Fettsäurealkanolamide verwendet werden, wobei die letzteren gleichzefig als Schaumstabilisatoren dienen.

### Stabilisatoren

Als Stabilisatoren können Metallsalze von Fettsäuren, wie z.B. Magnesium-, Aluminium- und/oder Zinkstearat bzw. -ricinoleat eingesetzt werden.

### Polymere

Geeignete kationische Polymere sind beispielsweise kationische Cellulosederivate, wie z.B. eine quatemierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quatemierte Kollagenpolypepfide, wie beispielsweise Lauryldimonium Hydroxypropyl Hydrolyzed Collagen (Lamequat®L/Grünau), quatemierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide, wie zB. beschrieben in der FR 2252840 A sowie deren vernetzte wasserlöslichen Polymere, kationische Chitinderivate wie beispielsweise quatemiertes Chitosan, gegebenenfalls mikrokristallin verteilt, Kondensationsprodukte aus Dihalogenalkylen, wie zB. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quatemierte Ammoniumsalz-Polymere, wie z.B. Mirapol® A-15, Mirapol® AD-1, Mirapole® AZ-1 der Firma Miranol.

Als anionische, zwitterionische, amphotere und nichtionische Polymere kommen beispielsweise Unylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvemetzte und mit Polyolen vernetzte Polyacrylsäuren, Acrylamidopropyltrimethylammoniumchlorid/ Acrylat-Copolymere, Octylacrylamid/Methylmeth-acrylat/tertButylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcapralactam-Terpolymere sowie gegebenenfalls derivatisierte Celluloseether und Silicone in Frage. Weitere geeignete Polymere und Verdickungsmittel sind in Cosm.Toll. 108,95 (1993**)** aufgeführt.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in Cosm.Toil. 91, 27 (1976**).**

### Biogene Wirkstoffe,

Unter biogenen Wirkstoffen sind im Rahmen der Erfindung zusätzlich solche zu verstehen, die nicht aus der Pflanze Cassia alata stammen, wie beispielsweise Tocopherolacetat, Tocopherolpalmitat, Ascorbinsäure, (Desoxy)Ribonucleinsäure und deren Fragmentierungsprodukte, Retinol, Bisabolol, Allantoin, Phytantriol, Panthenol, AHA-Säuren, Aminosäuren, Ceramide, Pseudoceramide, essentielle Öle, weitere Pflanzenextrakte und zusätzliche Vitaminkomplexe.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren. Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)hamstoff, 2,4,4'-Trichlor-2'-hydroxydiphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethyfester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von-Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butycyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiverol, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
➢ adstringierende Wirkstoffe,
➢ Ölkomponenten,
➢ nichtionische Emulgatoren,
➢ Coemulgatoren,
➢ Konsistenzgeber,
➢ Hilfsstoffe wie z. B. Verdicker oder Komplexierungsmittel und/oder
➢ nichtwässrige Lösungsmittel wie z. B. Ethanol, Propylenglykol und/oder Glycerid.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2, Aluminiumhydroxyallantoinat, Aluminiumchloridtartrat, Aluminium-Zirkonium-Trichlorohydrat, Aluminium-Zirko-nium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
➢ entzündungshemmende, hautschützende oder wohlriechende ätherische Öle,
➢ synthetische hautschützende Wirkstoffe und/oder
➢ öllösliche Parfümöle.

Übliche wasserlösliche Zusätze sind z.B. Konservierungsmittel, wasserlösliche Duftstoffe, pH-Wert Stellmittel, z.B. Puffergemische, wasserlösliche Verdickungsmittel, z.B. wasserlösliche natürliche oder synthetische Polymere wie - z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil.108, 95 (1993**)** entnommen werden.

### Insekten-Repellentien

Als Insekten-Repellentien kommen N,N-Diethyl-m-toluamid, 1,2-Pentandiol oder Ethyl Butylacetylaminopropionate in Frage

### Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
➢ Glyzerin;
➢ Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
➢ technische Oligoglyceringemische mit einem Eigenkondensationsgrad von 1,5 bis 10 wie etwa technische Diglyceringemische mit einem Diglyceringehalt von 40 bis 50 Gew.-%;
> Methyolverbindungen, wie insbesondere Trimethylolethan, Trimethylolpropan, Trimethylolbutan, Pentaerythrit und Dipentaerythrit;
➢ Niedrigalkylglucoside, insbesondere solche mit 1 bis 8 Kohlenstoffen im Alkylrest, wie beispielsweise Methyl- und Butylglucosid;
➢ Zuckeralkohole mit 5 bis 12 Kohlenstoffatomen, wie beispielsweise Sorbit oder Mannit,
➢ Zucker mit 5 bis 12 Kohlenstoffatomen, wie beispielsweise Glucose oder Saccharose;
➢ Aminozucker, wie beispielsweise Glucamin;
➢ Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen.

### Parfümöle

Als Parfümöle seien genannt Gemische aus natürlichen und synthetischen Riechstoffen. Natürlich Riechstoffe sind Extrakte von Blüten (Lilie, Lavendel, Rosert, Jasmin; Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalytbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöf, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

### Farbstoffe

Als Farbstoffe können die für kosmetische Zwecke geeigneten und zugelassenen Substanzen verwendet werden, wie sie beispielsweise in der Publikation **"**Kosmetische Färbemittel" der Farbstoffkommission der Deutschen Forschungsgemeinschaft, Verlag Chemie, Weinheim, 1984, S.81-106 zusammengestellt sind. Diese Farbstoffe werden üblicherweise in Konzentrationen von 0,001 bis 0,1 Gew.-%, bezogen auf die gesamte Mischung, eingesetzt.

### Beispiele

### Beispiel 1: Extraktion eines OPC- und Flavonoid-reichen Extrakts

100 g pulverförmige Litchi-Schale wurden in einem Becherglas mit 1000 ml Methanol versetzt und eine Stunde bei 50°C gerührt. Nach Filtration und Waschen des Rückstandes mit 100 ml Methanol wurde das Methanol der vereinigten Extrakte abdestilliert und der trockene Rückstand in 100 ml destilliertem Wasser aufgenommen. Nach Zentrifugation mit einer Cryofuge 6000 (15 Minuten bei 4200 U/min) wurde die wäßrige Lösung viermal mit jeweils 100 ml Essigester extrahiert. Die organische Phase wurde über 15 g Natriumsulfat getrocknet und der Essigester verdampft. Der Rückstand wurde wieder in destilliertem Wasser aufgenommen und viermal mit je 100 ml Essigester extrahiert. Die wässrige Lösung wurde nach abdestillieren des Essigesters lyophilisiert. Die Ausbeute betrug 6,13 g. Der Gehalt an OPC in dem so erhaltenen Extrakt wurde bestimmt nach der Methode von Porter et al. in Phytochemistry 25(1), pp 223-230, 1996: The conversion of procyanidins and prodelphinidins to cyanidin and delphinidin. Die Quantität an OPC in g bezogen auf 100 Extrakt betrug 19,98 %.

### Beispiel 2: Extraktion eines Rutin- und Anthocyan-reichen Extrakts

100 g pulverförmige Litchi-Schale wurden in einem Becherglas mit 1000 ml Methanol versetzt und eine Stunde bei 50 °C gerührt. Nach Filtration und Waschen des Rückstandes mit 100 ml Methanol wurde das Methanol der vereinigten Extrakte abdestilliert und der trockene Rückstand in 100 ml destilliertem Wasser aufgenommen. Nach Zentrifugation mit einer Cryofuge 6000 (15 Minuten bei 4200 U/min) wurde die wäßrige Lösung lyophilisiert.

### Beispiel 3: Zellschutzwirkung gegen UVA an in vitro gezüchteten menschlichen Fibroblasten

Hintergrund: UVA-Strahlen dringen bis in die Dermis ein, wo sie zu Oxidationsstreß führen, was durch eine Lipoperoxidation der Zytoplasmamembranen nachgewiesen wird.

Die Lipoperoxide werden zu Malonaldialdehyd (MDA) abgebaut, der viele biologische Moleküle wie Proteine und Nukleinbasen vernetzen wird (Enzymhemmung bzw. Mutagenese).

Methode: Zur Durchführung dieser Tests wurde ein definiertes Kulturmedium mit den Fibroblasten mit fötalem Kälberserum beimpft und der Pflanzenextrakt (in dem definierten Medium mit 10 % Fötalem Kälberserum) 72 Stunden nach dem Beimpfen zugegeben.

Nach 48 stündiger Inkubation bei 37 °C und einem CO₂-Gehalt von 5 % wurde das Kulturmedium durch Saline-Lösung (physiologische NaCl-Lösung) ersetzt und die Fibroblasten wurden mit einer UVA-Dosis bestrahlt (365 nm, 20 J/cm²; Röhren: MAZDA FLUOR TFWN40).

Nach der Beendigung der Bestrahlung wurde der MDA-Spiegel (Malonaldialdehyd-Spiegel) in der überstehenden Natriumchlorid-Lösung quantitativ durch Reaktion mit Thiobarbitursäure bestimmt.

**Tab.1: Bestrahlung von humanen Fibroblasten mit UVA (20 J/cm²) in vitro**

| **UVA-Bestrahlung** | **Angaben in % im Vergleich zur Kontrolle** | | |
|---|---|---|---|
| **20 J/cm²** | **Freigesetzter MDA** | **Zellproteine** | **GSH/Protein** |
| Kontrolle (nicht bestrahlt) | 0 | 100 | 100 |
| ohne Zusatz | 100 | 101 | 73 |
| 0,001 % Extrakt aus Beispiel 1 | 57 | 99 | 101 |
| 0,003 % Extrakt aus Beispiel 1 | 31 | 105 | 87 |
| 0,01 % Extrakt aus Beispiel 1 | 7 | 106 | 99 |
| 0,001 % Extrakte aus Beispiel 2 | 75 | 98 | 102 |
| 0,003 % Extrakt aus Beispiel 2 | 48 | 105 | 81 |
| 0,01 % Extrakt aus Beispiel 2 | 25 | 106 | 72 |
| 0,0003 % Tocopherol | 11 | 97 | 78 |

Die UVA-Bestrahlung hat zu einem starken Anstieg der Ausschüttung von MDA geführt, während sich der intrazelluläre GSH-Spiegel um ca. 27 % erniedrigte. Zusatz der Litchi-Fruchthüllen-Extrakte vermindert die Menge des freigesetzten MDA und hält den GSH-Spiegel auf hohem Niveau.

### Beispiel 4: Zeilschutzwirkung gegen UVB an in vitro gezüchteten menschlichen Keratinozyten

Hintergrund: UVB-Strahlen lösen durch Aktivierung eines Enzyms, nämlich Phospholipase A2 oder PLA2, das Arachidonsäure aus den Phospholipiden der Plasmamembran entfernt, eine Entzündung (Erythem, Ödem) aus. Arachidonsäure ist die Vorstufe der Prostaglandine, die eine Entzündung und eine Zellmembranschädigung verursachen; die Prostaglandine E2 (= PGE2) werden durch die Cyclooxygenase gebildet.

Methode: Der Effekt von UVB-Strahlung wurde an Keratinocyten in vitro untersucht indem die Freisetzung des Cytoplasaenzyms LDH (Lactat Dehydrogenase) bestimmt wurde. Dieses Enzym dient als Marker für eine Zellschädigung.

Zur Durchführung der Tests wurde ein definiertes Medium, das fötales Kälberserum enthält, mit den Keratinozyten beimpft und der Pflanzenextrakt (mit Saline-Lösung verdünnt) 72 Stunden nach dem Beimpfen zugegeben.

Die Keratinozyten wurden sodann mit einer UVB-Dosis bestrahlt (50 mJ/cm² - Röhren: DUKE FL40E).

Nach weiterer 1 tägiger Inkubation bei 37°C und bei 5 % CO₂ wurde der LDH-Gehalt im Überstand bestimmt. Der Gehalt von LDH- (Lactatdehydrogenase) wurde mittels einer Enzymreaktion bestimmt (verwendetes Kit zur Untersuchung des LDH Gehaltes von der Firma Roche). Die Anzahl adhärenter Keratinozyten wird (nach Trypsinbehandlung) mit einem Partikelzählgerät bestimmt.

**Tab. 2: Zeilschutzwirkung von Litchi-Fruchthüllen-Extrakten gegen UVB-Strahlen**

| **UVA-Bestrahlung** | **Angaben im % Vergleich zur Kontrolle** | |
|---|---|---|
| **50mJ/cm²** | **Anzahl Keratinocyten** | **freigesetztes LDH** |
| Kontrolle (nicht bestrahlt) | 100 | 0 |
| ohne Zusatz | 33 | 100 |
| 0,0001 % Extrakt aus Beispiel 1 | 37 | 90 |
| 0,0003 % Extrakt aus Beispiel 1 | 35 | 94 |
| 0,001 % Extrakt aus Beispiel 1 | 36 | 64 |
| 0,0003 % Extrakt aus Beispiel 2 | 36 | 87 |
| 0,001 % Extrakt aus Beispiel 2 | 35 | 84 |
| 0,003 % Extrakt aus Beispiel 2 | 34 | 73 |
| 0,001 % Aspirin | 33 | 68 |

Die UVB-Bestrahlung hat eine Erhöhung des LDH-Spiegels induziert, während die Zahl der lebensfähigen Keratinozyten um 67 % zurückgegangen ist. Bei Zusatz des Litchi-Fruchthüllen-Extraktes vermindert sich die Menge des freigesetzten LDH bis zu 25 %.

### Beispiel 5: Aktivität gegenüber freien Radikalen

In einer ersten Testreihe wurde die Eignung der Extrakte gegen oxidativen Stress untersucht Eingesetzt wurden die Extrakte gemäß der Beispiele 1 und 2.

Als erstes Testsubstrat wurde Diphenylpicrylhydrazyl (DPPH) gewählt, ein purpurrot gefärbtes stabiles Radikal, welches durch Inkontaktbringen mit Radikalfängern in sein ungefärbtes Leucoderivat übergeht. Der Farbwechsel kann photometrisch verfolgt werden. Die Meßergebnisse sind in Tabelle 3 zusammengefaßt ("DPPH-Test"). In einem weiteren Test wurde als Referenzsystem die Hydroxylierung von Salicylsäure durch Hydroxylradikale (aus der Reaktion von Wasserstoffperoxid mit Eisen(II)ionen und EDTA) untersucht. Auch diese Reaktion kann photometrisch untersucht werden, da das Hydroxylierungsprodukt rötlich gefärbt ist. Gemessen wurde der Einfluß der Extrakte auf die Bildung der Hydroxysalicylsäure bei einer optischen Dichte von 490 nm. Die Meßergebnisse sind ebenfalls in Tabelle 3 zusammengefaßt. In einem dritten und letzten Test wurde Xanthin Oxidase als Testsystem gewählt. Das Enzym bewirkt bei oxidativem Stress die Umwandlung von Purinbasen, wie z.B. Adenin oder Guanin in Uronsäure, wobei die intermediär gebildeten Sauerstoffradikale durch Reaktion mit Luminol über die Lumineszenz nachgewiesen und quantitativ bestimmt werden können. In Gegenwart von Substanzen mit radikalfangenden Eigenschaften vermindert sich die Lumineszenzausbeute. Diese Ergebnisse sind in Tabelle 4 zusammengefaßt; wieder ist die Inhibierung in %-absolut angegeben ("Lumina-Test").

**Tab. 3: Chemische Tests**

| EC50 in % | Litschi-Fruchthüllen Extrakt nach Beispiel 1 | Litschi-Fruchthüllen-Extrakt nach Beispiel 2 | Tocophenol | Ascorbinsäure |
|---|---|---|---|---|
| DPPH-Test | 0,001% | 0,0085% | 0,0067% | 0,0013% |
| Fenton's Reaktion | kein Effekt | 0,077% | nicht verfügbar | 0,1% |

**Tab. 4: Biochemische Tests**

| EC50 in % | Litchi-Fruchthüllen Extrakt nach Beispiel 1 | Litschi-Fruchthüllen Extrakt nach Beispiel 2 | Tocophenol | Ascorbinsäure |
|---|---|---|---|---|
| Luminol | 0,00006% | 0,00069% | Kein Effekt bis zu 1% | 0,0006% |
| Luminol + Microperoxydase | 0,00361 % | 0,02048% | Kein Effekt bis zu 1% | 0,0058% |
| NBT | 0,00836% | 0,04969% | Kein Effekt bis zu 1% | 0,5909% |

### Beispiel 6: Regenerierende und revitalisierende Aktivität auf der Haut

Das Ziel dieser Test ist der Nachweis einer regenerierenden und revitalisierenden Aktivität von Extrakten aus Litchi-Fruchthüllen an humanen Fibroblastenkulturen in vitro.

Humane Fibroblasten werden in einem definierten Nährmedium (DMEM = Dulbecco Minimum Essential Medium, Firma Life Technologie Sarl) mit 10 Gew. % fötalem Kälberserum angeimpft und für 24 h bei 37 °C in einer 5 %igen CO₂-Atmosphäre inkubiert. Anschließend wurde das Nährmedium mit fötalem Kälberserum durch ein Nährmedium aus DMEM ohne fötalem Kälberserum ausgetauscht. Zu diesem Nährmedium wurden unterschiedliche Konzentrationen an Aktivsubstanz in Form der beiden Extrakte der Fruchthülle von Litchi chinensis Sonn. gegeben. Nach einer dreitägigen Inkubation der Fibroblasten im Nährmedium wurde das Wachstum und die Stoffwechselaktivität beurteilt, indem der intrazelluläre Anteil an ATP nach der Methode von Vasseur (J. français Hydrologie, 1981, 9, 149-156) bestimmt wurde. Die Proteinmenge wurde nach der Methode von Bradford (Anal. Biochem., 1976, 72, 248-254) bestimmt.

**Tab 5: Toxizitätstest an humanen Fibroblasten**

| LD50 in % (w/v) | Litchi-Fruchthüllen-Extrakt nach Bsp. 1 | Litchi-Fruchthüllen-Extrakt nach Bsp. 2 | Tocopherol | Ascorbinsaure |
|---|---|---|---|---|
| Proteine | 0,015 | 0,023 | nicht toxisch bis 0,01 | 0,0055 |
| ATP | 0,005 | 0,019 | nicht toxisch bis 0,01 | 0,0056 |

Beide Litchi-Fruchthüllen-Extrakte haben den Zellmetabolismus nicht verbessert

### Beispiel 7: Inhibierung der Elastase-Aktivität

Serin-Proteasen, wie z.B. Elastase, bewirken den Abbau von Elastin, Proteoglycanen und Kollagen und verursachen damit eine Schwächung des Bindegewebes. Im folgenden Test wurden die inhibierenden Eigenschaften des Extraktes nach Beispiel 1 an einem chromogenen synthetischen Substrat (mit Kongorot markiert) untersucht. Die Inkubationszeit betrug 30 min bei Raumtemperatur. Die Inhibierung wurde photometrisch bei 410 nm verfolgt, als Positiv-Standard diente 1'α1-Antitrypsin. Die Ergebnisse sind in Tabelle 6 zusammengefaßt.

**Tabelle 6**

| **Extrakt nach Beispiel 1** | | |
|---|---|---|
| **Testsubstanz** | **Konzentration in % (W/v)** | **Elastase-Inhibierung in %** |
| Litchi-Extrakt nach Beispiel 1 | 3 | 60 |
| Litchi-Extrakt nach Beispiel 1 | 2 | 27 |
| 1'α1-Antitrypsin | 0,1 | 68 |

Ein 3%iger erfindungsgemäßer Extrakt aus den Fruchthüllen von Litchi chinensis Sonn. ist in der Lage, die Elastiase-Aktivität fast ebenso zu inhibieren wie der natürliche Inhibitor Antitrypsin.

### Beispiel 8: Inhibierung der Collagenase-Aktivität

Dermale Fibroblasten älterer Menschen schütten nach Sonnenexposition Collagenasen - auch Matrix Metallo-Protease (MMP) - aus. Im folgenden Test wurden die inhibierenden Eigenschaften des Extraktes nach Beispiel 1 an einem synthetischen Substrat MCA-Pro-Leu-Gly-Leu-DPA-Ala-Arg-NH₂ (Knight et al., 1992, FEBS Letter, 296, S. 263 - 266) und der menschlichen MMP-1 untersucht. Die Inkubationszeit betrug 60 min bei Raumtemperatur. Die Hydrolyse des Substrats wurde fluoreszenzspektrometrisch bei λₑₘ=393 nm (λₑₓ = 328 nm) bestimmt. Als Vergleichsstandard dient die Metallo-Protease TIMP-1 (natürlich im Gewebe vorkommender Inhibitor für MMP). Die Ergebnisse sind in Tab. 7 dargestellt.

**Tabelle 7**

| **Extrakt nach Beispiel 1** | | |
|---|---|---|
| **Testsubstanz** | **Konzentration in % (w/v)** | **MMP-1-Inhibierung in %** |
| Litchi Extrakt nach Beispiel 1 | 0,1 | 95 |
| Litchi Extrakt nach Beispiel 1 | 0,05 | 71 |
| Litchi Extrakt nach Beispiel 1 | 0,015 | 33 |
| Litchi Extrakt nach Beispiel 1 | 0,005 | 13 |
| TIMP-1 | 50 nMol | 93 |

Ein 0,1%iger erfindungsgemäßer Extrakt aus den Fruchthüllen von Litchi chinensis Sonn. ist in der Lage, die Collagenase-Aktivität fast ebenso zu inhibieren wie der natürliche Inhibitor TIMP-1.

### Beispiel 9: Inhibierung der Collagenase-Synthese

Man bedient sich dieses Tests zur Evaluierung der Fähigkeit des Litchi-Extraktes, die toxischen Effekte von UVA-Strahlung an in vitro kultivierten humanen Fibroblasten zu reduzieren. Hierzu wird sowohl die Menge des ausgeschütteten Enzyms MMP-1 (Matrix-Metallo-Proteinase) als auch die Menge des natürlichen Enzyminhibitors TIMP-1 bestimmt. Man bedient sich der UVA-Strahlung in diesem Test, da diese bis in die Dermis eindringen und oxidativen Stress induzieren kann, der zur Hautalterung führt.

Die Fibroblasten werden hierzu in einem genau definierten Medium mit fötalem Kälberserum kultiviert. Der Litchi-Extrakt nach Beispiel 1 wird 2 bis 3 Tage nach der Beimpfung zugefügt Nach einer weiteren Inkubationszeit von einem Tag bei 37 °C und einer CO₂-Konzentration von 5% wird das Kulturmedium gegen eine Salzlösung ersetzt und die Fibroblasten mit UVA bestrahlt (15 J/cm², Lampe: SOL500, Dr Höhnle, Filter. H1, Radiometer Vilbert Lourmat). Nach Beendigung der Bestrahlung werden die Fibroblasten weitere zwei Tage inkubiert. Der Gehalt des überstehenden Mediums an MMO-1 und TIMP-1 wird mit Hilfe der Untersuchungskits der Fa. Amersham (Kit Nr. RPN2610 und RPN2611) bestimmt. Der Kontrolle wurde kein Extrakt zugesetzt. Tabelle 8 faßt die erhaltenen Ergebnisse zusammen.

**Tab. 8: Nach UVA-Bestrahlung ausgeschüttetes MMP-1 und TIMP-1**

| **Zugesetze Substanz** | **ausgeschüttetes MMP-1 in ng/ml** | | | |
|---|---|---|---|---|
| | **ohne UVA-Bestrahlung** | | **nach UVA-Bestrahlung** | |
| | **Wert** | **Standardabweichung** | **Wert** | **Standardabweichung** |
| Kontrolle | 49 | 9 | 199 | 25 |
| Dexamethasone 0,1 µM | 2 | 0 | 7 | 3 |
| 0,0006 % Utcht-Extrakt nach Bsp. 1 | 60 | 20 | 140 | 17 |
| 0,003 % Utchi-Extrakt nach Bsp. | 82 | 11 | 164 | 8 |
| Kontrolle | 50 | 4 | 28 | 3 |
| Dexamethasone | 39 | 1 | 19 | 3 |
| 0,0006 % Litchi-Extrakt nach Bsp.1 | 53 | 1 | 15 | 2 |
| 0,003 % Utchi-Extrakt nach Bsp.1 | 56 | | 15 | 3 |
| 0,006 % Litchi-Extrakt nach Bsp.1 | 50 | 1 | 12 | 2 |

Die Menge des ausgeschütteten MMP-1 nach UVA-Bestrahlung wird deutlich vermindert.

### Beispiel 10: Inhibierung von Plasmin

Hintergrund: Plasmin ist eine menschliche Serin-Protease welche eine entscheidende Rolle in der Wundheilung hat. Es löst u.a. Fibrinklümpchen und unterstützt die Freisetzung von Keratinocyten welche zum Heilungsprozess beitragen.

Plasminogen stellt das Pro-Enzym dar, welches durch die Protease Urokinase zu Plasmin aktiviert wird. Die Urokinase wird von aktivierten Keratinocyten während der Wundheilung aber auch bei Hautirritationen oder kutanen Entzündungen sekretiert. Es konnte gezeigt werden, dass die Expression und die Sekretion von Urokinase durch den Einfluss von UVB-Strahlung auf der Haut induziert wird und dass das Pro-Enzym Plasminogen nahe der extrazellulären Matrix zu finden ist..

Plasmin ist des weiteren in der Lage pro-MMP3 zu aktivieren welches zur Verringerung von dermalen Glycoproteinen und Proteoglycanen beiträgt. Plasmin stellt dadurch eine wichtige Rolle bei Hautalterungsprozessen, speziell bei photo-induzierten Hautalterungsprozessen menschlicher Haut dar.

Methode: Humanes Plasmin wurde mit dem Extrakt gemäß Beispiel 1 gemischt und für 5 min. bei 20 °C inkubiert. Anschließend wurde entweder synthetisches Substrat, speziell "Val-Leu-paranitroanilide" von der Firma Chromogenix oder natürliches pro-MMP3, bezogen von Merck-Eurolab, dem Gemisch zugesetzt. Über einen Zeitraum von 30 min. wurde bei dem synthetischen Substrat alle 5 min. die Absorption bei 405 nm untersucht und damit die Freisetzung von Paranitroaniline bestimmt. Mit Hilfe der Western-blot Methode nach einer 6-stündigen Inkubation bei 37 °C konnte die enzymatische Aktivität in der zweiten Testreihe nachgewiesen werden.

Die Inhibierung der enzymatischen Aktivität wurde gegen eine Kontrolle ohne Substrat und gegen eine Referenzsubstanz, speziell Aprotinine von der Firma Sigma getestet

**Tab. 9: Plasmininhibierung nach Zugabe des synthetischen Substrates**

| **Litchi Extrakt nach Beispiel 1 [µg/ml]** | **% Inhibierung im Vergleich zur Kontrolle** |
|---|---|
| 0 | 0 |
| 2.5 | 0 |
| 5 | 55 |
| 10 | 65 |
| 25 | 83 |
| 50 | 90 |
| 100 | 94 |
| Aprotinine: 50 µg/ml | 100 |

Bei einer Konzentration von 4,5 µg/ml des Litchi-Extraktes gemäß Beispiel 1 liegt eine 50 %ige Inhibierung vor.

**Tab.10: Inhibierung der Aktivierung von pro-MMP3 durch Plasmin**

| | **Gehalt an pro-MMP3** |
|---|---|
| Kontrolle ohne Plasmin | 49260 |
| Kontrolle mit Plasmin | 1435 |
| Litchi Extrakt nach Beispiel 1 - 0.003 % | 1117 |
| Litchi Extrakt nach Beispiel 1- 0.03 % | 44641 |

### Beispiel 11: Anti-Inflammatorische Aktivität

Hintergrund: Eine kutane Inflammation (Entzündung) kann hervorgerufen werden durch UVB Strahlung aufgrund der Stimulierung epidermaler Keratinocyten. Anschließend setzt eine akute Leukozyten Infiltration ein.

Diese Aktivierung dieser Leukozyten, speziell polymorphonucleare neutrophile Granulocyten, ist bekannt als "respiratory burst" und kann eine Gewebezerstörung vermitteln durch freigesetzten reaktiven Sauerstoffradikalen (reactive oxygen species - ROS) und durch lysosomale Enzyme.

Methode: Die anti-inflammatorische Aktivität wurde an einer Zelllinien humaner Leukocyten (neutrophile Granulocyten) untersucht. Zu diesem Zweck wurden die Zellen mit unterschiedlichen Konzentrationen an zu testenden Extrakten gemäß Beispiel 1 inkubiert und anschließend ein "respiratory burst" durch Hefezellenextrakt ("Zymosan") induziert indem 0,1 ml Zymosan 30 min. die Zellen aktivierten. Der Anteil an intermediär gebildeten Sauerstoffradikale wurde durch Reaktion mit Luminol über die emittierte Lumineszenz über 60 sec. nachgewiesen und quantitativ bestimmt. In Gegenwart von Substanzen mit radikalfangenden Eigenschaften vermindert sich die Lumineszenzausbeute. Diese Ergebnisse sind in Tabelle 4 zusammengefasst; der Anteil an freigesetzten Radikalen wurde gegen eine Referenzsubstanz (Minocycline - ein Radikalfänger) ermittelt. Die Ergebnisse sind angegeben in % relativ zur Kontrolle. Zur Kontrolle wurde jeweils die Anzahl der intakten Zellen mit einem Partikelzähler bestimmt und in % im Vergleich zur Kontrolle angegeben.

**Tab.11: Bestimmung der freigesetzten Sauerstöffradikale**

| Substanz | Konzentration | Anteil ROS (%/Kontrolle) | Anzahl intakter Zellen |
|---|---|---|---|
| Kontrolle | | 100 | 100 |
| Minocycline (Sigma) 0,001 % | | 28 | 100 |
| Extrakt nach Beispiel 1 | 0,0001 | 92 | 99 |
| | 0,001 | 25 | 96 |

Aus den obigen Ergebnissen lässt sich entnehmen, dass die erfindungsgemäßen Extrakte nach Beispiel 1 bei einer Konzentration von 0,001 % einen starken anti-inflammatorischen Effekt zeigen.

### Beispielrezepturen kosmetischer Mittel

Die gemäß Beispiel 1 erhaltenen Extrakte wurden in den folgenden erfindungsgemäßen eingesetzt. Die so hergestellten kosmetischen Mittel zeigten gegenüber den Vergleichsrezepturen V1, V2 und V3 sehr gute hautpflegende Eigenschaften bei gleichzeitig guter Hautverträglichkeit. Darüber hinaus sind die erfindungsgemäßen Mittel stabil gegen oxidative Zersetzung.

**Tabelle 12: Softcreme Rezepturen K1 bis K7**

| (Alle Angaben in Gew.-% bezogen auf das kosmetische Mittel) | | | | | | | | |
|---|---|---|---|---|---|---|---|---|
| INCI Bezeichnung | K1 | K2 | K3 | K4 | K5 | K6 | K7 | V1 |
| Glyceryl Stearate (and) | 8,0 | 8,0 | 8,0 | 8,0 | 8,0 | 8,0 | 8,0 | 8,0 |
| Ceteareth-12120 (and) Cetearyl | | | | | | | | |
| Alcohol (and) Cetyl Palmitate | | | | | | | | |
| Cetearyl Alcohol | 2,0 | 2,0 | 2,0 | 2,0 | 2,0 | 2,0 | 2,0 | 2,0 |
| Dicaprylyl Ether | 2,0 | 2,0 | 2,0 | 2,0 | 2,0 | 2,0 | 2,0 | 2,0 |
| Cocoglycerides | 3,0 | 3,0 | 3,0 | 3,0 | 3,0 | 3,0 | 3,0 | 3,0 |
| Cetearyl Isononanoate | 3,0 | 3,0 | 3,0 | 3,0 | 3,0 | 3,0 | 3,0 | 3,0 |
| Glycerin (86 Gew.-%ig) | 3,0 | 3,0 | 3,0 | 3,0 | 3,0 | 3,0 | 3,0 | 3,0 |
| Extrakt nach Beispiel 1 | 0,5 | 0,5 | 0,5 | 0,5 | 0,5 | 0,5 | 0,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | Ad 100 | | | | | | | |

**Tabelle 13: Nachtcremerezepturen K8 bis K14**

| (Alle Angaben in Gew.-% bezogen auf das kosmetische Mittel) | | | | | | | | |
|---|---|---|---|---|---|---|---|---|
| INCI Bezeichnung | K8 | K9 | K10 | K11 | K12 | K13 | K14 | V2 |
| Polyglyceryl-2 | 4,0 | 4,0 | 4,0 | 4,0 | 4,0 | 4,0 | 4,0 | 5,0 |
| Dipolyhydroxystearate | | | | | | | | |
| Polyglyceryl-3 Diisostearate | 2,0 | 2,0 | 2,0 | 2,0 | 2,0 | 2,0 | 2,0 | 2,0 |
| Cera Alba | 2,0 | 2,0 | 2,0 | 2,0 | 2,0 | 2,0 | 2,0 | 2,0 |
| Zinc Stearate | 2,0 | 2,0 | 2,0 | 2,0 | 2,0 | 2,0 | 2,0 | 2,0 |
| Cocoglycerides | 3,0 | 3,0 | 3,0 | 3,0 | 3,0 | 3,0 | 3,0 | 3,0 |
| Cetaeryl Isononanoate | 8,0 | 8,0 | 8,0 | 8,0 | 8,0 | 8,0 | 8,0 | 8,0 |
| Dicaprylyl Ether | 5,0 | 5,0 | 5,0 | 5,0 | 5,0 | 5,0 | 5,0 | 5,0 |
| Magnesiumsulfate | 1,0 | 1,0 | 1,0 | 1,0 | 1,0 | 1,0 | 1,0 | 1,0 |
| Glycerin (86 Gew: %ig) | 5,0 | 5,0 | 5,0 | 5,0 | 5,0 | 5,0 | 5,0 | 5,0 |
| Extrakt nach Beispiel 1 | 0,5 | 0,5 | 0,5 | 0,5 | 0,5 | 0,5 | 0,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | Ad 100 | | | | | | | |

**Tabelle 14: W/O Bodylotion Rezepturen K15 bis K21**

| (Alle Angaben in Gew.-% bezogen auf das kosmetische Mittel) | | | | | | | | |
|---|---|---|---|---|---|---|---|---|
| INCI-Bezeichnung | K15 | K16 | K17 | K18 | K19 | K20 | K21 | V3 |
| PEG-7 Hydrogenated | 7,0 | 7,0 | 7,0 | 7,0 | 7,0 | 7,0 | 7,0 | 7,0 |
| Castor Oil | | | | | | | | |
| Decyl Oleate | 7,0 | 7,0 | 7,0 | 7,0 | 7,0 | 7,0 | 7,0 | 7,0 |
| Cetearyl Isononanoate | 7,0 | 7,0 | 7,0 | 7,0 | 7,0 | 7,0 | 7,0 | 7,0 |
| Glycerin (86 Gew.-%ig) | 5,0 | 5,0 | 5,0 | 5,0 | 5,0 | 5,0 | 5,0 | 5,0 |
| MgSO₄ * 7 H₂O | 1,0 | 1,0 | 1,0 | 1,0 | 1,0 | 1,0 | 1,0 | 1,0 |
| Extrakt nach Beispiel 1 | 1,5 | 1,5 | 1,5 | 1,5 | 1,5 | 1,5 | 1,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | Ad 100 | | | | | | | |

| | | | | | | | | |
|---|---|---|---|---|---|---|---|---|
| ¹⁾ Desoxyribonucleinsäure: Molekuargewicht ca. 70000, Reinheit (bestimmt durch spektrophotometrische Messung der Absorption bei 260 nm sowie 280 nm): mindestens 1,7. | | | | | | | | |

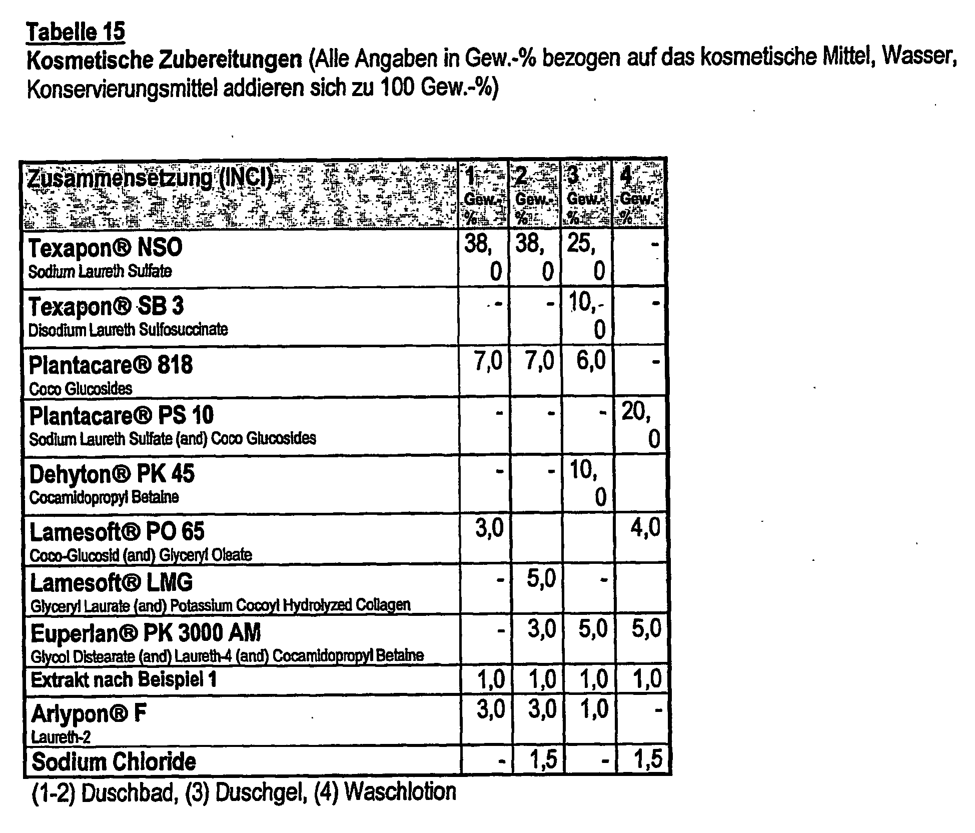

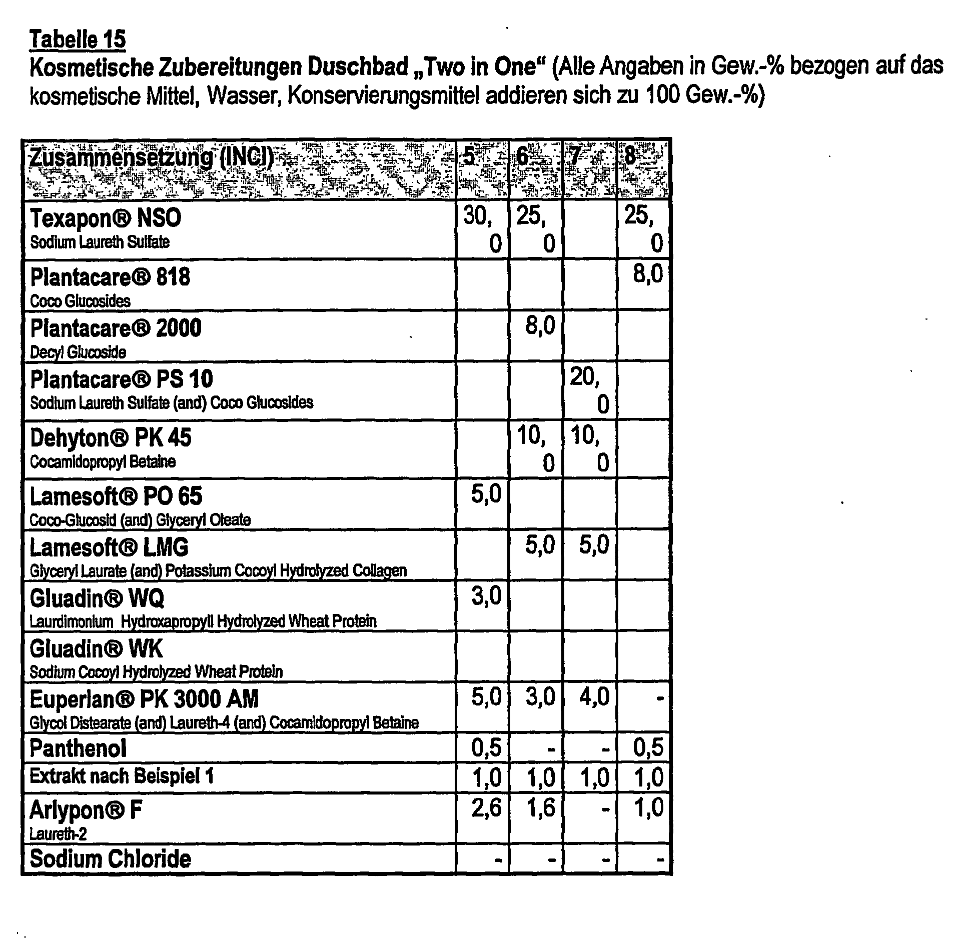

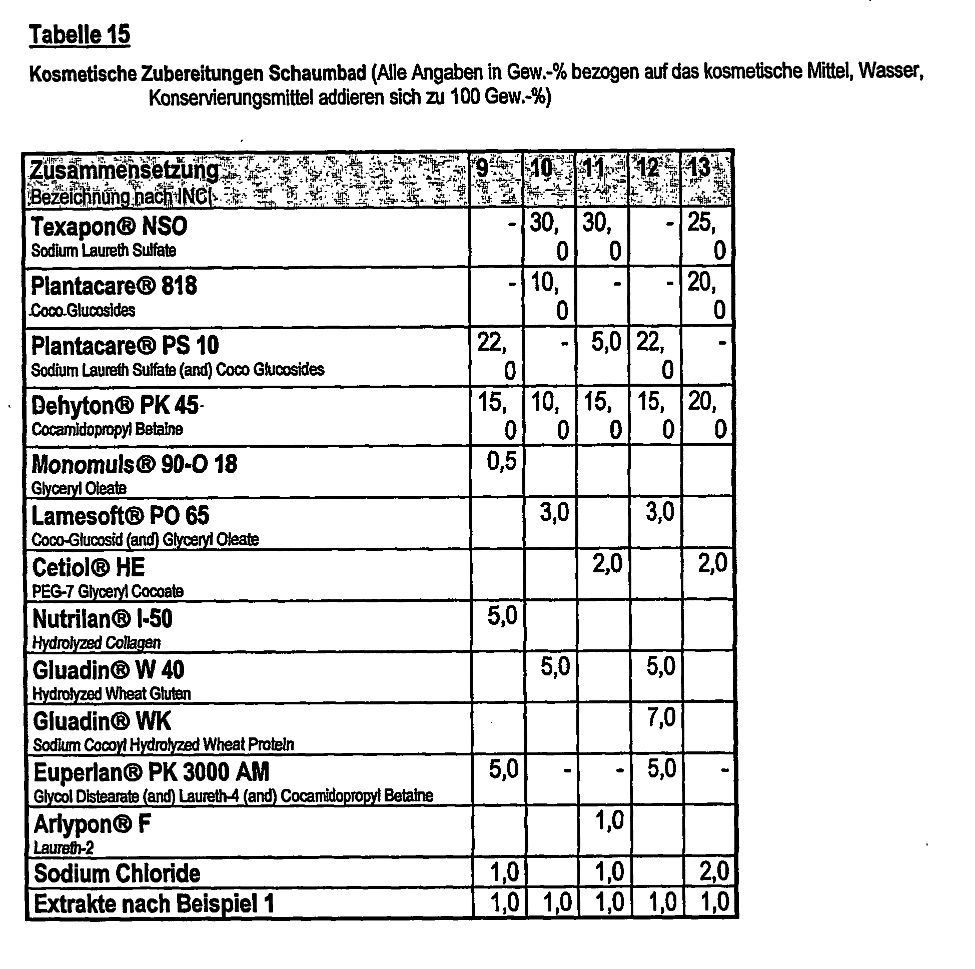

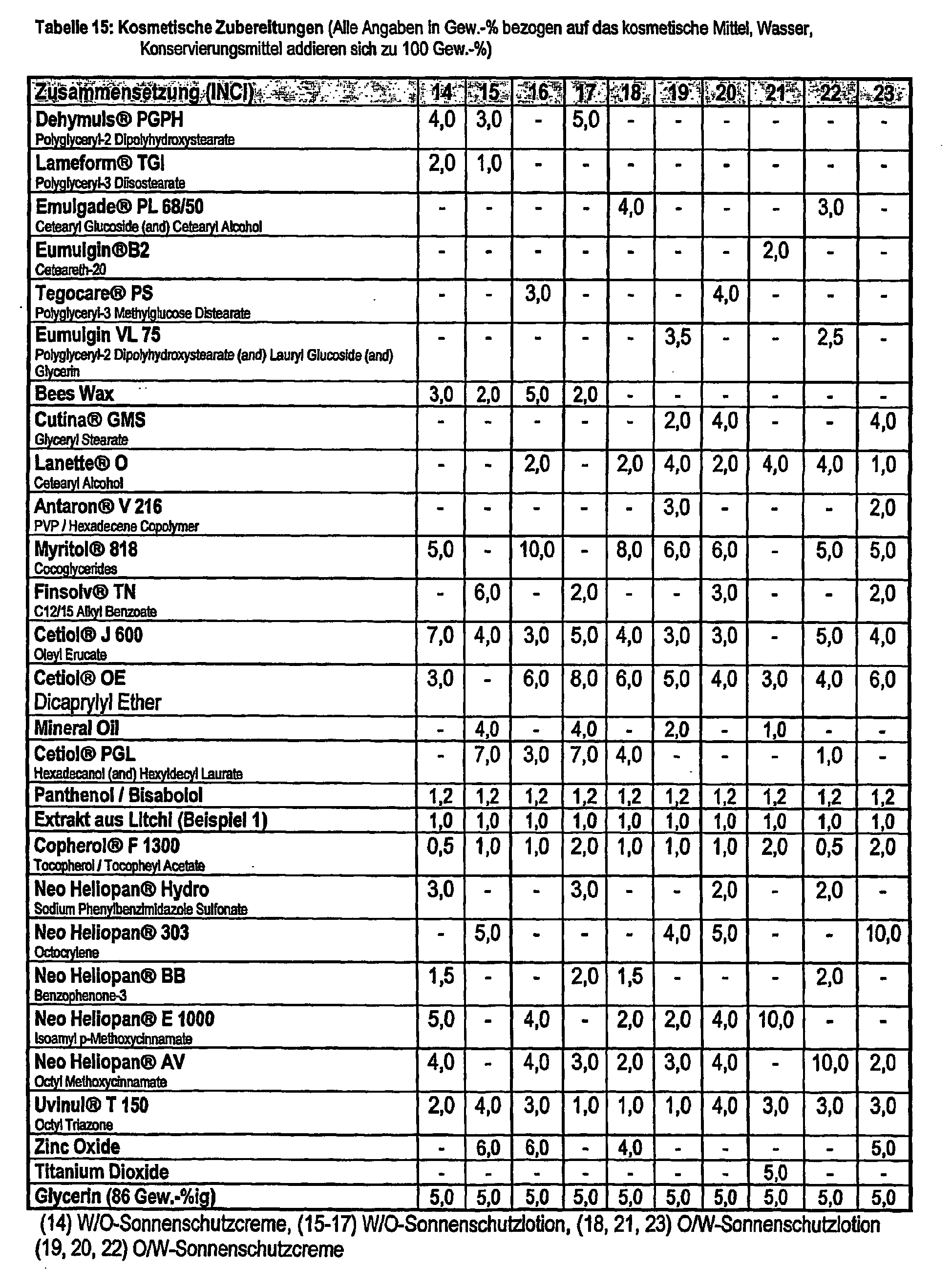

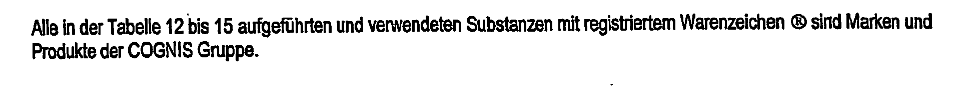

## Patentansprüche

1. Kosmetische Verwendung von Extrakten aus der Fruchthülle der Pflanze Litchi chinensis Sonn. als Pflegemittel für Haut und/oder Haare, wobei die Extrakte Flavonderivate enthalten, die ausgewählt sind aus der Gruppe, die gebildet wird von Flavanen, Flavan-3-olen, Flavan-3,4-diolen, Flavonen, Flavonolen, Flavononen und deren Derivate.

2. Kosmetische Verwendung von Extrakten aus der Fruchthülle der Pflanze Litchi chinensis Sonn. als Sonnenschutzmittel, insbesondere gegen UVA- und/oder gegen UVB-Strahlung, wobei die Extrakte Flavonderivate enthalten, die ausgewählt sind aus der Gruppe, die gebildet wird von Flavanen, Flavan-3-olen, Flavan-3,4-diolen, Flavonen, Flavonolen, Flavononen und deren Derivate.

3. Kosmetische Verwendung von Extrakten aus der Fruchthülle der Pflanze Litchi chinensis Sonn. gegen freie Radikale, wobei die Extrakte Flavonderivate enthalten, die ausgewählt sind aus der Gruppe, die gebildet wird von Flavanen, Flavan-3-olen, Flavan-3,4-diolen, Flavonen, Flavonolen, Flavononen und deren Derivate.

4. Kosmetische Verwendung von Extrakten aus der Fruchthülle der Pflanze Litchi chinensis Sonn. als Antioxidans, wobei die Extrakte Flavonderivate enthalten, die ausgewählt sind aus der Gruppe, die gebildet wird von Flavanen, Flavan-3-olen, Flavan-3,4-diolen, Flavonen, Flavonolen, Flavononen und deren Derivate.

5. Kosmetische Verwendung von Extrakten aus der Fruchthülle der Pflanze Litchi chinensis Sonn. als anti-inflammatorische Mittel, insbesondere zur Inhibierung der schädlichen Effekte von "respiratory burst" aufgrund des Durchdringens von Polymorphonuclearen Leukocyten in gestresster Haut, wobei die Extrakte Flavonderivate enthalten, die ausgewählt sind aus der Gruppe, die gebildet wird von Flavanen, Flavan-3-olen, Flavan-3,4-diolen, Flavonen, Flavonolen, Flavononen und deren Derivate.

6. Kosmetische Verwendung von Extrakten aus der Fruchthülle der Pflanze Litchi chinensis Sonn. gegen die Hautalterung, wobei die Extrakte Flavonderivate enthalten, die ausgewählt sind aus der Gruppe, die gebildet wird von Flavanen, Flavan-3-olen, Flavan-3,4-diolen, Flavonen, Flavonolen, Flavononen und deren Derivate.

7. Kosmetische Verwendung von Extrakten aus der Fruchthülle der Pflanze Litchi chinensis Sonn. als Protease-inhibierendes Mittel, insbesondere als Plasmin(Serin-Protease)-inhibierendes Mittel und/oder als MMP-und/oder Collagenase- und/oder Elastase-inhibierendes Mittel, wobei die Extrakte Flavonderivate enthalten, die ausgewählt sind aus der Gruppe, die gebildet wird von Flavanen, Flavan-3-olen, Flavan-3,4-diolen, Flavonen, Flavonolen, Flavononen und deren Derivate.

## Claims

1. Cosmetic use of extracts from the pericarp of the plant Litchi chinensis Sonn. as skin-care and/or hair-care agent, the extracts containing flavone derivatives selected from the group consisting of flavans, flavan-3-ols, flavan-3,4-diols, flavones, flavonols, flavonones and derivatives thereof.

2. Cosmetic use of extracts from the pericarp of the plant Litchi chinensis Sonn. as sun protection factor, more particularly against UV-A and/or UV-B radiation, the extracts containing flavone derivatives selected from the group consisting of flavans, flavan-3-ols, flavan-3,4-diols, flavones, flavonols, flavonones and derivatives thereof.

3. Cosmetic use of extracts from the pericarp of the plant Litchi chinensis Sonn. against free radicals, the extracts containing flavone derivatives selected from the group consisting of flavans, flavan-3-ols, flavan-3,4-diols, flavones, flavonols, flavonones and derivatives thereof.

4. Cosmetic use of extracts from the pericarp of the plant Litchi chinensis Sonn. as antioxidant, the extracts containing flavone derivatives selected from the group consisting of flavans, flavan-3-ols, flavan-3,4-diols, flavones, flavonols, flavonones and derivatives thereof.

5. Cosmetic use of extracts from the pericarp of the plant Litchi chinensis Sonn. as anti-inflammatory, more particularly for inhibiting the harmful effects of respiratory burst caused by the penetration of polymorphonuclear leucocytes in stressed skin, the extracts containing flavone derivatives selected from the group consisting of flavans, flavan-3-ols, flavan-3,4-diols, flavones, flavonols, flavonones and derivatives thereof.

6. Cosmetic use of extracts from the pericarp of the plant Litchi chinensis Sonn. against ageing of the skin, the extracts containing flavone derivatives selected from the group consisting of flavans, flavan-3-ols, flavan-3,4-diols, flavones, flavonols, flavonones and derivatives thereof.

7. Cosmetic use of extracts from the pericarp of the plant Litchi chinensis Sonn. as protease inhibitor, more particularly as plasmin (serine protease) inhibitor and/or as MMP and/or collagenase and/or elastase inhibitor, the extracts containing flavone derivatives selected from the group consisting of flavans, flavan-3-ols, flavan-3,4-diols, flavones, flavonols, flavonones and derivatives thereof.

## Revendications

1. Utilisation cosmétique d'extraits de l'enveloppe du fruit de la plante Litchi chinensis Sonn. comme agent de soin pour la peau et/ou les cheveux, les extraits contenant des dérivés de flavone, qui sont choisis dans le groupe formé par les flavanes, les flavan-3-ols, les flavane-3,4-diols, les flavones, les flavonols, les flavonones et leurs dérivés.

2. Utilisation cosmétique d'extraits de l'enveloppe du fruit de la plante Litchi chinensis Sonn. comme agent de protection solaire, en particulier contre le rayonnement UVA et/ou UVB, les extraits contenant des dérivés de flavone, qui sont choisis dans le groupe formé par les flavanes, les flavan-3-ols, les flavane-3,4-diols, les flavones, les flavonols, les flavonones et leurs dérivés.

3. Utilisation cosmétique d'extraits de l'enveloppe du fruit de la plante Litchi chinensis Sonn. contre les radicaux libres, les extraits contenant des dérivés de flavone, qui sont choisis dans le groupe formé par les flavanes, les flavan-3-ols, les flavane-3,4-diols, les flavones, les flavonols, les flavonones et leurs dérivés.

4. Utilisation cosmétique d'extraits de l'enveloppe du fruit de la plante Litchi chinensis Sonn. comme antioxydant, les extraits contenant des dérivés de flavone, qui sont choisis dans le groupe formé par les flavanes, les flavan-3-ols, les flavane-3,4-diols, les flavones, les flavonols, les flavonones et leurs dérivés.

5. Utilisation cosmétique d'extraits de l'enveloppe du fruit de la plante Litchi chinensis Sonn. comme agent anti-inflammatoire, en particulier pour inhiber les effets nuisibles de la stimulation du métabolisme oxydatif ("respiratory burst"), en raison de la pénétration de leucocytes polymorphonucléaires dans la peau stressée, les extraits contenant des dérivés de flavone, qui sont choisis dans le groupe formé par les flavanes, les flavan-3-ols, les flavane-3,4-diols, les flavones, les flavonols, les flavonones et leurs dérivés.

6. Utilisation cosmétique d'extraits de l'enveloppe du fruit de la plante Litchi chinensis Sonn. contre le vieillissement de la peau, les extraits contenant des dérivés de flavone, qui sont choisis dans le groupe formé par les flavanes, les flavan-3-ols, les flavane-3,4-diols, les flavones, les flavonols, les flavonones et leurs dérivés.

7. Utilisation cosmétique d'extraits de l'enveloppe du fruit de la plante Litchi chinensis Sonn. comme agent inhibant les protéases, en particulier comme agent inhibant la plasmine (sérine-protéase) et/ou comme agent inhibant la MMP et/ou la collagénase et/ou l'élastase, les extraits contenant des dérivés de flavone, qui sont choisis dans le groupe formé par les flavanes, les flavan-3-ols, les flavane-3,4-diols, les flavones, les flavonols, les flavonones et leurs dérivés.
